Europäisches Patentamt

(19) European Patent Office

Office européen des brevets

(11) Publication number : **0 224 721 B1**

## (12) EUROPEAN PATENT SPECIFICATION

(45) Date of publication of patent specification :
05.06.91 Bulletin 91/23

(21) Application number : 86115034.0

(22) Date of filing : 29.10.86

(51) Int. Cl.$^5$ : **C07G 1/00**, D21C 3/20

(54) **Recovery of lignin.**

(30) Priority : 05.11.85 US 795069

(43) Date of publication of application :
10.06.87 Bulletin 87/24

(45) Publication of the grant of the patent :
05.06.91 Bulletin 91/23

(84) Designated Contracting States :
AT BE CH DE ES FR GB IT LI NL SE

(56) References cited :
US-A- 2 331 154
US-A- 3 223 697
US-A- 3 585 104
TAPPI JOURNAL, vol. 68, no. 8, August 1985, pages 94-97, Norcross, Georgia, US; J.H. LORA et al.: "Organosolv pulping: a versatile approach to wood refining"

(73) Proprietor : **Repap Technologies Inc.**
**2650 Eisenhower Avenue**
**Valley Forge, PA 19482 (US)**

(72) Inventor : **Lora, Jairo H.**
**450 Forrest Avenue**
**Norristown PA 19401 (US)**
Inventor : **Katzen, Raphael**
**2868 Alpine Terrace**
**Cincinnati OH 45208 (US)**
Inventor : **Cronlund, Malcolm**
**434 S. Saddlebrook Circle**
**Chester-Springs PA 19425 (US)**
Inventor : **Wu, Chih Fae**
**Apt. C 291 Murray Drive**
**King of Prussia PA 19406 (US)**

(74) Representative : **Patentanwälte Grünecker,**
**Kinkeldey, Stockmair & Partner**
**Maximilianstrasse 58**
**W-8000 München 22 (DE)**

## Description

## BACKGROUND OF THE INVENTION

This invention relates to a process for precipitating lignin from a solution of lignin in a water miscible organic solvent, and to an apparatus for carrying out the process.

Processes for treating wood with organic solvents, such as alcohols, to separate the wood's lignin, hemicellulose, sugar and cellulose fractions are now well known, see, for example, U.S. patent 1,856,567 and U.S. Patent 3,585,104. Such solvent pulping processes have appeared to be attractive alternatives to conventional chemical pulping processes, such as kraft and sulfite pulping processes which suffer from relatively high equipment costs and pollution problems.

One solvent pulping process, disclosed in U.S. Patent 4,100,016, has appeared to be particularly attractive in providing highly efficient recovery of its alcohol solvent, separation of the cellulose and lignin fractions of wood, and recovery of cellulose pulp with no appreciable air or water pollution or solid waste products. This patented process has also provided hardwood pulps with yields, strengths, Kappa numbers, viscosities, fiber strengths and bleachability characteristics that are equal to or better than kraft and sulfite hardwood pulps.

However, the recovery of lignin from the alcohol/water extract or "black liquor", produced as a by-product of the solvent pulping process of U.S. Patent 4,100,016, has been relatively inefficient and difficult to control. Lignin has been recovered from the black liquor in this patent by first stripping (preferably vacuum stripping) alcohol from the black liquor and then separating the lignin which precipitates from the stripper bottoms or tails (preferably by thickening and then centrifuging the settled solids from the stripper bottoms). However, a portion of the lignin has tended to precipitate as a sticky tar or gum on the internal surfaces of the stripper, thereby fouling the stripper and reducing its efficiency in recovering alcohol from the black liquor. The lignin also has tended to precipitate from the stripper bottoms as a sticky amorphous mass which has been difficult to handle and has required substantial crushing to convert the lignin mass into a powder.

As a result, more efficient ways have been sought for removing lignin from the black liquor produced by a solvent pulping process such as is disclosed in U.S. Patent 4,100,016. One method has involved precipitating lignin from the alcohol/water black liquor by diluting it with water, see Rydholm, "Pulping Processes", pp. 672-673. Interscience Publishers, New York (1971). However, this method has resulted in very slow settling rates of the lignin, and in some cases, a very stable collodial suspension of the lignin has been formed which has been difficult to filter or centrifuge.

Tappi Journal, Vol. 68, No. 8, pages 94-97 describes the fractionation of hardwoods into pulp, lignin, and hemicellulose in a multi-stage alcohol extraction pilot plant by Organasolv Pulping. Lignin is obtained as a dry powder having a number average molecular weight of about 1000 and a polydispersity of about 3.

## SUMMARY OF THE INVENTION

An aim of the invention is to provide a relatively simple way of recovering lignin from an alcohol/water black liqour in high yields and high rates in an easy to handle and useful form.

This is achieved, in accordance with the invention, by a process for precipitating lignin from a solution of lignin in a water miscible organic solvent, characterized by diluting the lignin-containing solution with water and an acid to form a diluted aqueous solution with : a pH of less than 3, an organic solvent content of less than 30% (by volume), and a temperature of less than 75°C.

The lignin, which precipitates from the diluted aqueous solution, can be dried to a powder which requires little or no crushing to convert it into a fine uniform size suitable for use without further significant processing.

The lignin product of the above process, preferably has a number average molecular weight of 800 to 1500 g/mol and a polydispersity of less than 4.

Further provided, in accordance with another aspect of the invention, is an apparatus for carrying out the process by precipitating lignin from, a black liquor, that is, an apparatus for pulping wood or other fibrous plant material with a water miscible organic solvent to produce a black liquor as a by-product and for subsequently recovering the solvent from the black liquor, characterized by :

a) means for diluting the black liquor with an aqueous acid to precipitate lignin from the resulting diluted black liquor and leave a lignin-depleted supernatant ;

b) means for thereafter separating the precipitated lignin from the lignin-depleted supernatant;

c) means for thereafter removing the organic solvent from the lignin-depleted supernatant to form a solvent- and lignin-depleted supernatant ; and

d) means for thereafter recycling a portion of the solvent- and lignin-depleted supernatant to the diluting means for use as the aqueous acid.

## BRIEF DESCRIPTION OF THE DRAWINGS

Fig. 1 is a flow chart of a process for producing cellulose pulp from wood by treating the wood with an aqueous alcohol solvent and for recovering lignin from the alcohol/water black liquor that is a by-product of the process.

Fig. 2 is a schematic sectional view of an example of an apparatus for precipitating lignin from the alcohol/water black liquor from the process of Fig. 1.

## DETAILED DESCRIPTION OF THE PREFERRED EMBODIMENTS

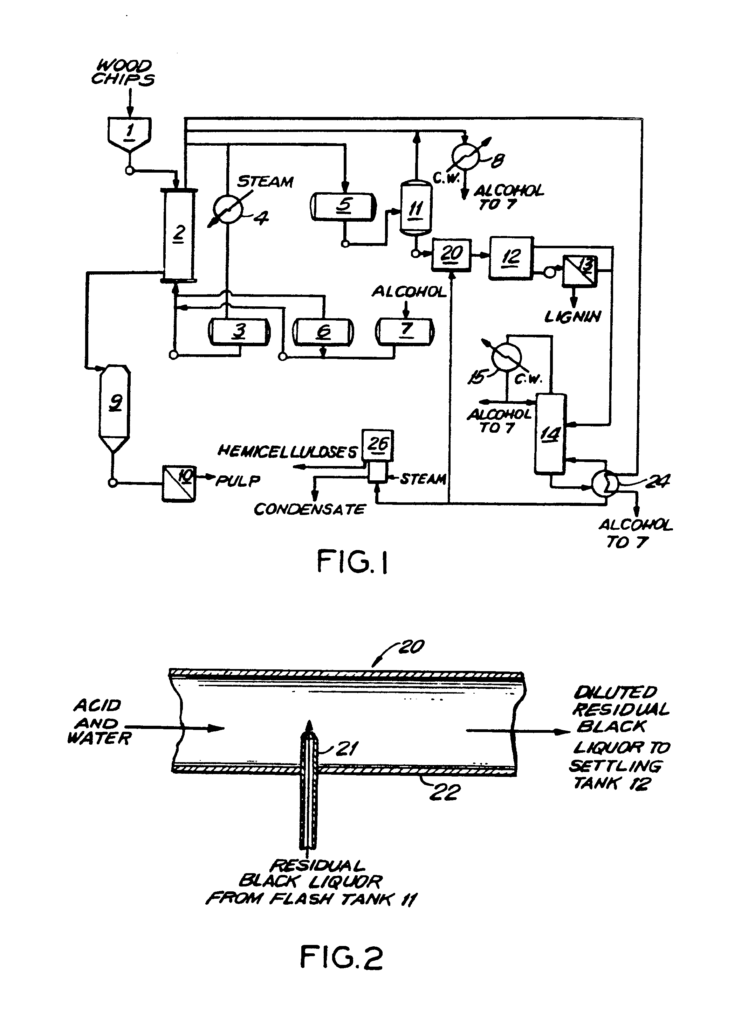

The process shown in Fig. 1 initially involves pulping a batch of wood chips that are loaded from a hopper 1 into an extractor 2. The extractor 2 is operated in accordance with the aforesaid U.S. Patent 4,100,016 at an elevated temperature (e.g., 180 to 210°C) and an elevated pressure (e.g., 20 to 35 atmospheres) and with a solvent comprising : 40 to 80% (by volume) of a water miscible lower aliphatic alcohol of 1 to 4 carbon atoms (e.g., methanol, ethanol, isopropanol or tert-butanol) ; 20 to 60% water ; and if needed, a small amount of a strong water soluble acid, such as a mineral acid (e.g., hydrochloric, sulfuric, phosphoric or nitric acid) or an organic acid (e.g., oxalic acid).

Preferably, the wood chips in the extractor 2 are preheated with low pressure steam, and then, a twice-used 60% ethanol/40% water, primary solvent from a primary solvent accumulator 3 contacts the wood chips in the extractor 2. The primary solvent is rapidly recirculated through the extractor 2 and through a peak load (e.g., steam-heated) heat exchanger 4 to raise the temperature of the wood chips to 190 to 200°C in a few (preferably not more than 5) minutes. After this first pulping step is completed, the resulting extract or "black liquor" in the extractor 2 is displaced into a recovery feed accumulator 5 by a once-used 60% ethanol/40% water, secondary solvent (preferably heated to 190 to 200°C) from a secondary solvent accumulator 6. At the end of this displacement, the secondary solvent in the extractor 2 is displaced into the primary solvent accumulator 3 by a fresh 60% ethanol/40% water solvent (preferably heated to 190 to 200°C) from a fresh solvent accumulator 7. The fresh solvent in the extractor 2 is then drained into the secondary solvent accumulator 6. Once the extractor 2 has been drained, it is vented, alcohol-rich vapors from the extractor are condensed in a water-cooled ("C.W.") condensor 8, and the resulting ethanol/water mixture from the condensor 8 is recycled to the fresh solvent accumulator 7. After venting the extractor 2, residual alcohol in the pulp in the extractor is then stripped with low pressure steam, and the resulting alcohol/water vapors are condensed and recovered as discussed below. After steam stripping, the pulp in the extractor 2 is sluiced with water, piped to a holding tank 9 and pumped through a pulp screen 10. The pulp can then be suitably subjected to conventional pulp handling, bleaching and paper-making procedures. The extractor 2 can be loaded with another batch of wood chips from the hopper 1, and the wood chips can be contacted by the primary, secondary and fresh solvents from accumulators 3, 6 and 7 as described above.

The lignin, hemicelluloses, other saccharides and extractives (e.g., resins, organic acids, phenols and tannins) from the wood and the ethanol, which are all present in the black liquor at a temperature of 180 to 210°C and under a pressure of 20.2 to 35.4 bars (20 to 35 atmospheres) in the recovery feed accumulator 5, are then recovered by first flashing the black liquor into a flash tank 11 to recover part of the ethanol. The flash tank 11 can be at atmospheric pressure for simplicity of operation or at reduced pressure to further cool the black liquor and enhance the alcohol reovery. The reduction in pressure in the flash tank 11 causes partial vaporization of the ethanal and leaves the residual black liquor in the flash tank with an ethanol content of 30 to 45%, preferably 35 to 40%. The residual black liquor is cooled during this step to a temperature of less than 95°, preferably down to 80 to 92°, but not below 70° to avoid premature precipitation of lignin in the flash tank 11. The ethanol/water vapors obtained are condensed and recycled, along with any make-up ethanol, water and/or acid, to the fresh solvent accumulator 7 for use in treating subsequent batches of wood chips.

Lignin is then separated from the residual black liquor, discharged from the flash tank 11, by the process of this invention. This step is carried out by diluting and preferably cooling the residual black liquor, as it leaves the flash tank 11, with water and acid to form a diluted residual black liquor with : a) an alcohol content of less than 30% (by volume), preferably 10 to 25%, particularly 12 to 21%, with an alcohol content of 8% being a practical minimum for subsequently recovering the alcohol economically ; b) a temperature of less than 75°C, preferably less than 60°C, particularly 35 to 55°C ; and c) a pH of less than 3, preferably less than 2.5, particularly 1.5 to 2.5. In this step, particular temperatures are not critical, although providing higher temperatures in the diluted residual black liquor will generally increase settling rates of the lignin but will yield a darker colored lignin and may decrease its yields. 75°C is a maximum temperature to avoid the formation of tarry lignin precipitates, and ambient temperature (e.g., 20°C) is a practical minimum, although lower temperatures (e.g., down to 0°C) can be used if low settling rates can be tolerated. Temperatures below 65°C, particularly below 60°C, provide a significantly lighter colored lignin precipitate. Also, particular pH's of the diluted residual black liquor are not critical in this step, but lower pH's increase the yield of precipitated lignin from the diluted residual black liquor and permit the use of higher temperatures in the diluted residual black liquor. However, lowering pH below 1 provides little or no additional improvement in yield, and for this reason, a pH of 1 is a practical minimum although lower pH's can be used. At a pH of less than 3, lignin will precipitate from the residual black liquor in high yield and at a high rate as fine solids. These lignin solids can then be separated from the remaining diluted residual black liquor supernatant in a conventional manner. Preferably, the lignin solids are separated by : allowing them to settle out as a paste of 6 to 12% (by weight) solids in a conventional clarifier or settling tank 12 ; then concentrating this paste of lignin solids in a conventional centrifugal separator 13 to form a wet cake of 30 to 40% solids ; and then drying this wet cake to form a uniform fine, free flowing powder.

In diluting the residual black liquor from the flash tank 11 with the water and acid to precipitate lignin, any conventional water soluble acid can be utilized which will provide the diluted residual black liquor with a pH of less than 3.0, preferably less than 2.50. For example, a strong mineral acid (e.g., hydrochloric, nitric, sulfuric or phosphoric acid) or a strong organic acid (e.g., oxalic acid) can be used. Preferably, the water and acid are mixed together before they are used to dilute the residual black liquor. In this regard, a particularly preferred mixture of acid and water is a residual black liquor supernatant that is derived from a previous batch of wood chips and that has been recycled and used to dilute the residual black liquor from the flash tank 11 after : a) the supernatant has been separated from the lignin solids from the previous batch of wood chips in the settling tank 12 and the centrifugal separator 13 ; and b) the alcohol content of the supernatant has been recovered in a conventional solvent recovery tower 14 provided with a conventional solvent condenser 15 as described below. This recycled residual black liquor supernatant or stripper bottoms, when used for diluting the residual black liquor from the flash tank 11, provides higher yields and faster settling of lignin solids precipitating in the settling tank 12 and centrifugal separator 13.

In precipitating lignin from the residual black liquor from the flask tank 11, the method of diluting the residual black liquor with the water and acid also is not critical, so long as there is rapid and intimate mixing of the residual black liquor with the acid and water. For example, the residual black liquor can be suitably diluted by adding it to the acid and water in a conventional static dispersion mixer. The residual black liquor can also be diluted by adding it as a finely divided stream to a stream comprising a solution of the water and acid, for example, by means of a venturi-type device, generally 20, as shown schematically in Fig. 2. The residual black liquor from flash tank 11 in Fig. 1 can be pumped through a small nozzle 21 located at about the center of a pipe 22 in the venturi-type device 20 in Fig. 2, and the acid and water solution can flow in the pipe 22 towards the settling tank 12 in Fig. 1. As the residual black liquor is injected by the nozzle 21 into the acid and water solution in the pipe 22, the residual black liquor is rapidly diluted and cooled by the acid and water in the pipe 22. Lignin rapidly precipitates as fine solids from the resulting diluted residual black liquor in the pipe 22, which solids can be easily collected and concentrated in the settling tank 12 and centrifugal separator 13.

In precipitating lignin in accordance with the above-described embodiment of the process of this invention, the yield and settling rates of the lignin are generally a function of : a) the wood species ; b) the process conditions utilized in the extractor 2 ; c) the temperature, pH and solids content of (i) the residual black liquor from the flash tank 11 and (ii) the acid and water used to dilute it ; and d) the ratio of residual black liquor to the acid and water used to dilute it. For example, the lignin from softwoods, such as spruce, is preferably precipitated at a temperature after dilution of 40 to 60°C using an acid and water solution with a pH of 1.5 to 2.5 and with a ratio of residual black liquor to the acid and water solution of 0.5 to 1. For hardwoods such as aspen, it is preferred to use an acid and water solution with a pH of 1.2 to 2.2 and a temperature after dilution of less than 50°C. In this regard, it is preferred to use a ratio of residual black liquor to the acid and water solution of : a) 0.2 to 0.8 if the temperature after dilution is above 40°C ; and b) 0.6 to 1.0 if the temperature after dilution is less than 40°C (e.g., down to ambient temperature). For hardwoods, such as sweetgum, maple and oak, it is preferred to use a temperature after dilution of 40 to 60°C, an acid and water solution with a pH of 1.5 to 2.5, and a ratio of residual black liquor to the acid and water solution of 0.35 to 0.7.

As shown in Fig. 1, the ethanol content of the clarified residual black liquor supernatant from the settling tank 12 and centrifugal separator 13 is preferably recovered in the solvent recovery tower 14 and solvent condenser 15. The ethanol content of the supernatant can be stripped (e.g., down to 200 ppm) in a conventional manner in the solvent recovery tower 14 at atmospheric pressure. Preferably, the tower 14 is heated by heating and recycling a portion of the bottoms stream from the tower 14 in a heat exchanger 24, using the low pressure steam used to strip residual ethanol from the pulp in the extractor 2. The ethanol/water vapors from the tower 14 are condensed in a conventional manner in the water-cooled condenser 15 (or by heat exchange with the stripper feed) and are then recycled to the fresh solvent accumulator 7 together with the ethanol/water mixture which condenses from the low pressure steam in the heat exchanger 24. The ethanol content of the supernatant from the settling tank 12 and centrifugal separator 13 can be suitably recovered in high yield in a simple manner, without lignin precipitating within the solvent recovery tower 14 and forming tarry or gummy deposits on the internal surfaces of the tower.

A portion of the bottoms stream removed from the solvent recovery tower 14 is preferably concentrated in a conventional manner, for example, in multiple effect evaporators 26. In this step, scaling or fouling of the evaporation equipment is not a significant problem because there are no substantial amounts of high molecular weight lignin in the bottoms stream from the solvent recovery tower 14. The resulting syrup, containing hemicelluloses together with small amounts of other saccharides, extractives and very low molecular weight lignin (i.e., lignin with a molecular weight of less than 400 g/mol), can be burned to recover its fuel value, used as animal feed, or converted to other chemical products.

A second portion of the bottoms stream removed from the tower 14 is preferably used as the acid and water solution for diluting the residual black liquor from the flash tank 11 in order to precipitate lignin therefrom. In this regard, the second portion of the bottoms stream from the tower 14 is preferably cooled to a temperature of less than 50°C, preferably 25 to 40°C (about 0°C being a practical minimum), and its pH is adjusted, if necessary, to 1.0 to 3.0 by adding a strong water soluble acid to it. Then, the cooled and acidified second portion of the bottoms stream (hereinbefore called the "recycled residual black liquor supernatant") is intimately and rapidly mixed (e.g., in the venturi-type device 20 of Fig. 2) with the residual black liquor to dilute and cool the residual black liquor and precipitate lignin.

The very pure lignin, which precipitates as fine solids from the diluted residual black liquor in the settling tank 12, can be subsequently removed from the centrifugal separator 13, water-washed and dried in a conventional manner (e.g., by spin flash drying) to form a fine (e.g., –80 mesh) uniform, free flowing, water insoluble powder. This lignin can be characterized as having : a relatively low number average molecular weight of 800 to 1500 g/mol, preferably 900 to 1300 g/mol ; a narrow molecular weight distribution, i.e., a polydispersity of less than 4, preferably no more than 3, particularly only 1.5 to 2.7 ; and a methoxyl content approximately equal to the methoxyl content of native lignin (i.e., 20% for hardwoods and 14% for softwoods). This lignin also has a glass transition temperature which is preferably 100 to 170°, particulaly 130 to 150°. These characteristics show, inter alia, the purity and low degree of chemical modification of the lignin as obtained by the process of this invention. This lignin can be used, for example, as a phenol formaldehyde resin extender in the manufacture of particle board and plywood. This lignin can also be used in the manufacture of molding compounds, urethane and epoxy resins, antioxidants, controlled-release agents and flow control agents.

The process, product and apparatus aspects of this invention and many of their attendant advantages will be understood from, the foregoing description, and it will be apparent that various modifications and changes can be made in the process and apparatus for precipitating lignin without departing from the scope of these aspects of the invention of sacrificing all of their material advantages. The process and apparatus hereinbefore described being merely preferred embodiments. For example, the process of this invention can alternatively be carried out by separately adding an acid and water to a solution of lignin dissolved in a water miscible organic solvent to form a diluted aqueous solution with a pH of less than 3, an organic solvent content of les than 30% and a temperature of less than 75°C, from which diluted solution the lignin will precipitate as uniform fine solids. In this regard, the acid can be separately added to the residual black liquor from the flash tank 11 in Fig. 1 by adding the acid to the primary solvent from the primary solvent accumulator 3 before the primary solvent is used in the extractor 2 for pulping wood chips to produce the black liquor (which becomes, after removal of ethanol in the flash tank 11, the residual black liquor). Also, the process of this invention can be carried out with a water miscible organic solvent other than a lower aliphatic alcohol (preferably ethanol), such as acetone, glycol or glycerol, or with a mixture of such solvents. Also, this process can be carried out with lignin extracted from any fibrous plant material, such as bamboo, bagasse, and cereal straws, and not just wood.

## Claims

1. A process for precipitating lignin from a solution of lignin in a water miscible organic solvent, characterised by diluting the lignin-containing solution with water and an acid to form a diluted aqueous solution with a pH of less than 3, an organic solvent content of less than 30% by volume and a temperature of less than 75°C.

2. The process according to claim 1, characterised by providing the organic solvent content of the diluted aqueous solution in the form of a lower aliphatic alcohol of 1 to 4 carbon atoms.

3. The process according to claim 2, characterised by forming the diluted aqueous solution with a pH of less than 2.5.

4. The process according to claim 3, characterised by forming the diluted aqueous solution with a temperature of less than 60°C.

5. The process according to claim 4, characterised by forming the diluted aqueous solution with a temperature of 35 to 55°C.

6. The process according to claim 2, characterised by forming the diluted aqueous solution with an alcohol content of 10 to 25% by volume.

7. The process according to claim 6, characterised by forming the diluted aqueous solution with an alcohol content of 12 to 21% by volume and with a temperature of less than 65°C.

8. The process according to claim 6, characterised by forming the diluted aqueous solution with a temperature of less than 60°C and a pH of 1.5 to 2.5.

9. The process according to claim 8, characterised by forming the diluted aqueous solution with a temperature of 35 to 55°C and alcohol content of 12 to 21% by volume.

10. The process according to claim 1, characterised by using, as the water for diluting the lignin-containing solution, water which is in addition to water already present in the solution.

11. The process according to claim 10, characterised by intimately and rapidly mixing the lignin-containing solution with a mixture of the acid and the additional water to form the diluted aqueous solution.

12. The process according to claim 1, characterised by utilising, as the lignin-containing solution, a solution obtained by contacting wood or other fibrous plant material with the organic solvent at an elevated temperature and an elevated pressure to produce a cellulose pulp and a black liquor containing lignin and the organic solvent, and then separating the pulp from the black liquor.

13. The process according to claim 12, characterised by utilising the acid and water in a mixture which also contains hemicelluloses, other saccharides, extractives and lignin with a molecular weight of less than 400 g/mol.

14. The process according to claim 13, characterised by utilising, as the acid and water mixture, a recycled aqueous acid obtained by diluting the black liquor with the aqueous acid to form a diluted black liquor from which lignin is precipitated, leaving a black liquor supernatant; removing the organic solvent from the black liquor supernatant to produce a residual black liquor supernatant; and then recycling a portion of the residual black liquor supernatant for use as the aqueous acid in diluting the black liquor.

15. The process according to claim 14, characterised by utilising, as the organic solvent, a lower aliphatic alcohol of 1 to 4 carbon atoms.

16. The process according to claim 15, characterised by providing the diluted black liquor with a pH of less than 2.5.

17. The process according to claim 16, characterised by providing the diluted black liquor with a temperature of less than 60°C.

18. The process according to claim 15, characterised by providing the diluted black liquor with an alcohol content of 10 to 25% by volume.

19. The process according to claim 18, characterised by providing the diluted black liquor with a pH of 1.5 to 2.5 and a temperature of less than 60°C.

20. The process according to claim 19, characterised by providing the diluted black liquor with a temperature of 35 to 55°C and an alcohol content of 12 to 21% by volume.

21. The process according to claim 14, characterised by the fact that the black liquor contains water before it is diluted with the recycled aqueous acid.

22. The process according to claim 21, characterised by the fact that the black liquor is intimately and rapidly mixed with the recycled aqueous acid to form the diluted black liquor.

23. The process according to claim 15, characterised by providing the diluted black liquor with a temperature of 70 to 95°C and the recycled aqueous acid with a temperature of less than 50°C.

24. The process according to claim 23, characterised by providing the diluted black liquor with a temperature of 80 to 92°C and the recycled aqueous acid with a temperature of 25 to 40°C.

25. An apparatus for pulping wood or other fibrous plant material with a water miscible organic solvent to produce a black liquor as a by-product and for subsequently recovering the solvent from the black liquor, characterised by :

a) means for diluting the black liquor with an aqueous acid to precipitate lignin from the resulting diluted black liquor and leave a lignin-depleted supernatant ;

b) means for thereafter separating the precipitated lignin from the lignin-depleted supernatant;

c) means for thereafter removing the solvent from the lignin-depleted supernatant to form a solvent- and lignin-depleted supernatant ; and

d) means for thereafter recycling a portion of the solvent- and lignin-depleted supernatant to the diluting means for use as the aqueous acid.

26. The apparatus according to claim 25, characterised by the fact that the diluting means is associated with means for removing a portion of the solvent from the black liquor before diluting the black liquor.

27. The apparatus according to claim 26, characterised by the fact that the recycling means is associated with means for adding a strong water soluble acid to the solvent- and lignin-depleted supernatant before recycling it to the diluting means.

28. The apparatus according to claim 25, characterised by the fact that the diluting means includes an arrangement for intimately and rapidly mixing the black liquor with the aqueous acid.

## Ansprüche

1. Verfahren zum Ausfällen von Lignin aus einer Lösung von Lignin in einem mit Wasser mischbaren organischen Lösungsmittel, dadurch gekennzeichnet, daß man die Lignin enthaltende Lösung mit Wasser und einer Säure verdünnt zur Bildung einer verdünnten wäßrigen Lösung mit einem pH von weniger als 3, einem Gehalt an organischem Lösungsmittel von weniger als 30 Vol.-% und einer Temperatur von weniger als 75°C.

2. Verfahren nach Anspruch 1, dadurch gekennzeichnet, daß man den Gehalt an organischem Lösungsmittel der verdünnten wäßrigen Lösung in Form eines niederen aliphatischen Alkohols mit 1 bis 4 Kohlenstoffatomen vorsieht.

3. Verfahren nach Anspruch 2, dadurch gekennzeichnet, daß man die verdünnte wäßrige Lösung mit einem pH von weniger als 2,5 bildet.

4. Verfahren nach Anspruch 3, dadurch gekennzeichnet, daß man die verdünnte wäßrige Lösung mit einer Temperatur von weniger als 60°C bildet.

5. Verfahren nach Anspruch 4, dadurch gekennzeichnet, daß man die verdünnte wäßrige Lösung mit einer Temperatur von 35 bis 55°C bildet.

6. Verfahren nach Anspruch 2, dadurch gekennzeichnet, daß man die verdünnte wäßrige Lösung mit einem Alkoholgehalt von 10 bis 25 Vol.-% bildet.

7. Verfahren nach Anspruch 6, dadurch gekennzeichnet, daß man die verdünnte wäßrige Lösung mit einem Alkoholgehalt von 12 bis 21 Vol.-% und mit einer Temperatur von weniger als 65°C bildet.

8. Verfahren nach Anspruch 6, dadurch gekennzeichnet, daß man die verdünnte wäßrige Lösung mit einer Temperatur von weniger als 60°C und einem pH von 1,5 bis 2,5 bildet.

9. Verfahren nach Anspruch 8, dadurch gekennzeichnet, daß man die verdünnte wäßrige Lösung mit einer Temperatur von 35 bis 55°C und einem Alkoholgehalt von 12 bis 21 Vol.-% bildet.

10. Verfahren nach Anspruch 1, dadurch gekennzeichnet, daß man als Wasser zum Verdünnen der Lignin enthaltenden Lösung Wasser verwendet, das zu dem bereits in der Lösung vorhandenen Wasser zusätzlich hinzukommt.

11. Verfahren nach Anspruch 10, dadurch gekennzeichnet, daß man die Lignin enthaltende Lösung gründlich und rasch mit einer Mischung aus der Säure und dem zusätzlichen Wasser mischt zur Bildung der verdünnten wäßrigen Lösung.

12. Verfahren nach Anspruch 1, dadurch gekennzeichnet, daß man als Lignin enthaltende Lösung eine Lösung verwendet, die erhalten wird, durch Kontaktieren von Holz oder einem anderen fasrigen Pflanzenmaterial mit dem organischen Lösungsmittel bei erhöhter Temperatur und erhöhtem Druck, um eine Cellulosepulpe und eine Schwarzlauge zu erzeugen, die Lignin und das organische Lösungsmittel enthält, und Abtrennen der Pulpe von der Schwarzlauge.

13. Verfahren nach Anspruch 12, dadurch gekennzeichnet, daß man die Säure und Wasser in einer Mischung verwendet, die ebenso Hemicellulosen, andere Saccharide, Extraktionsmittel und Lignin mit einem Molekulargewicht von weniger als 400 g/Mol enthält.

14. Verfahren nach Anspruch 13, dadurch gekennzeichnet, daß man als die Säure- und Wassermischung eine rezyklierte wäßrige Säure verwendet, die erhalten wird durch Verdünnen der Schwarzlauge mit der wäßrigen Säure, um eine verdünnte Schwarzlauge zu bilden, aus der Lignin ausgefällt wird, Zurücklassen einer überstehenden Schwarzlauge ; Entfernen des organischen Lösungsmittels aus der überstehenden Schwarzlauge, um eine restliche überstehende Schwarzlauge zu erzeugen ; und danach Rezyklisieren eines Teils der restlichen überstehenden Schwarzlauge zur Verwendung als wäßrige Säure beim Verdünnen der Schwarzlauge.

15. Verfahren nach Anspruch 14, dadurch gekennzeichnet, daß man als organisches Lösungsmittel einen niederen aliphatischen Alkohol mit 1 bis 4 Kohlenstoffatomen verwendet.

16. Verfahren nach Anspruch 15, dadurch gekennzeichnet, daß man die verdünnte Schwarzlauge mit einem pH von weniger als 2,5 vorsieht.

17. Verfahren nach Anspruch 16, dadurch gekennzeichnet, daß man die verdünnte Schwarzlauge mit einer Temperatur von weniger als 60°C vorsieht.

18. Verfahren nach Anspruch 15, dadurch gekennzeichnet, daß man die verdünnte Schwarzlauge mit einem Alkoholgehalt von 10 bis 25 Vol.-% vorsieht.

19. Verfahren nach Anspruch 18, dadurch gekennzeichnet, daß man die verdünnte Schwarzlauge mit einem pH von 1,5 bis 2,5 und einer Temperatur von weniger als 60°C vorsieht.

20. Verfahren nach Anspruch 19, dadurch gekennzeichnet, daß man die verdünnte Schwarzlauge mit einer Temperatur von 35 bis 55°C und einem Alkoholgehalt von 12 bis 21 Vol.-% vorsieht.

21. Verfahren nach Anspruch 14, gekennzeichnet durch den Umstand, daß die Schwarzlauge Wasser enthält, bevor sie mit der rezyklierten wäßrigen Säure verdünnt wird.

22. Verfahren nach Anspruch 21, gekennzeichnet durch den Umstand, daß die Schwarzlauge gründlich und rasch mit der rezyklierten wäßrigen Säure vermischt wird zur Bildung der verdünnten Schwarzlauge.

23. Verfahren nach Anspruch 15, dadurch gekennzeichnet, daß man die verdünnte Schwarzlauge mit einer Temperatur von 70 bis 95°C und die rezyklierte wäßrige Säure mit einer Temperatur von weniger als 50°C vorsieht.

24. Verfahren nach Anspruch 23, dadurch gekennzeichnet, daß man die verdünnte Schwarzlauge mit einer Temperatur von 80 bis 92°C und die rezyklierte wäßrige Säure mit einer Temperatur von 25 bis 40°C vorsieht.

25. Vorrichtung zum Aufschließen von Holz oder anderem fasrigen Pflanzenmaterial mit einem mit Wasser mischbaren organischen Lösungsmittel, um eine Schwarzlauge als Nebenprodukt zu erzeugen und zum nachfolgenden Gewinnen des Lösungsmittels aus der Schwarzlauge, gekennzeichnet durch :

a) Mittel zum Verdünnen der Schwarzlauge mit einer wäßrigen Säure, um Lignin aus der resultierenden verdünnten Schwarzlauge auszufällen und eine an Lignin verarmte überstehende Flüssigkeit zurückzulassen ;

b) Mittel zum darauffolgenden Abtrennen des ausgefällten Lignins aus der an Lignin verarmten überstehenden Flüssigkeit ;

c) Mittel zum darauffolgenden Entfernen des Lösungsmittels aus der an Lignin verarmten überstehenden Flüssigkeit zur Bildung einer an Lösungsmittel und Lignin verarmten überstehenden Flüssigkeit ; und

d) Mittel zum darauffolgenden Rezyklisieren eines Teils der an Lösungsmittel und Lignin verarmten überstehenden Flüssigkeit zu dem Verdünnungsmittel zur Verwendung als die wäßrige Säure.

26. Vorrichtung nach Anspruch 25, gekennzeichnet durch den Umstand, daß das Verdünnungsmittel verbunden ist mit einem Mittel zum Entfernen eines Teils des Lösungsmittels aus der Schwarz lauge vor dem Verdünnen der Schwarzlauge.

27. Vorrichtung nach Anspruch 26, gekennzeichnet durch den Umstand, daß das Mittel zum Rezyklisieren verbunden ist mit einem Mittel für die Zugabe einer starken wasserlöslichen Säure zu der an Lösungsmittel und Lignin verarmten überstehenden Flüssigkeit vor deren Rezyklisierung zu dem Verdünnungsmittel.

28. Vorrichtung nach Anspruch 25, gekennzeichnet durch den Umstand, daß das Mittel zum Verdünnen eine Anordnung beinhaltet für das gründliche und rasche Mischen der Schwarzlauge mit der wäßrigen Säure.


**Revendications**

1. Un processus industriel pour précipiter de la lignine à partir d'une solution composée de lignine dans un solvant organique miscible dans l'eau et qui consiste à diluer cette solution de lignine avec de l'eau et de l'acide afin d'obtenir une solution aqueuse diluée dont le pH est inférieur à 3, le volume de solvant organique inférieur à 30% par volume et la température de moins de 75°C.

2. Conformément à la revendication 1, ce procédé consiste à utiliser un alcool gras composé seulement de 1 à 4 atomes de carbone comme solvant organique de la solution aqueuse diluée.

3. Conformément à la revendication 2, ce procédé consiste à donner un pH de moins de 2,5 à la solution aqueuse diluée.

4. Conformément à la revendication 3, ce procédé consiste à donner une température inférieure a 60°C à la solution aqueuse diluée.

5. Conformément a la revendication 4, ce procédé consiste à donner une température de 35 à 55°C à la solution aqueuse diluée.

6. Conformément à la revendication 2, ce procédé consiste à donner une teneur en alcool de 10 à 25% par volume à la solution aqueuse diluée.

7. Conformément à la revendication 6, ce procédé consiste à donner une teneur en alcool de 12 à 21% par volume et une température inférieure à 65°C à la solution aqueuse diluée.

8. Conformément à la revendication 6, ce procédé consiste à donner une température inférieure 60°C, et pH de 1,5 à 2,5 à la solution aqueuse diluée.

9. Conformément à la revendication 8, ce procédé consiste à donner une température de 35 à 55°C et une teneur en alcool de 12 à 21% par volume à la solution aqueuse diluée.

10. Conformément à la revendication 1, ce procédé consiste à ajouter de l'eau à celle que contient la solution de lignine afin de diluer cette solution.

11. Conformément a la revendication 10, ce procédé consiste à mélanger intimement et rapidement la solution de lignine avec une mixture de l'acide et de l'eau ajoutée afin d'obtenir la solution aqueuse diluée.

12. Conformément à la revendication 1, ce procédé consiste à utiliser une solution produite en mettant en contact du bois ou toute autre plante fibreuse avec le solvant organique à une température et une pression élevées afin de produire une pâte de cellulose et un liquide noir contenant de la lignine et le solvant organique, puis à séparer la pâte de ce liquide noir pour obtenir la solution de lignine.

13. Conformément à la revendication 12, ce procédé consiste à mettre l'acide et l'eau dans un mélange contenant également des hémicelluloses, d'autres saccharides, des extractifs et de la lignine d'un poids moléculaires inférieur à 400 g/mole.

14. Conformément à la revendication 13, ce procédé consiste à utiliser, comme mélange d'eau et d'acide, un acide aqueux recyclé obtenu par dilution du liquide noir avec l'acide aqueux et aboutissant ainsi à un liquide noir surnatant après précipitation de la lignine qu'il contient ; à extraire le solvant organique du liquide noir surnantant afin d'obtenir un liquide noir surnatant résiduel ; ensuite une partie du liquide noir résiduel et surnatant est retraitée et sera utilisée comme l'acide aqueux employé pour diluer le liquide noir.

15. Conformément à la revendication 14, ce procédé consiste à utiliser comme solvant organique un alcool gras composé seulement de 1 à 4 atomes de carbone.

16. Conformément à la revendication 15, ce procédé consiste à donner un pH de moins de 2,5 au liquide noir dilué.

17. Conformément à la revendication 16, ce procédé consiste à donner une température inférieure à 60°C au liquide noir dilué.

18. Conformément à la revendication 15, ce procédé consiste à donner une teneur en alcool de 10 à 25% par volume au liquide noir dilué.

19. Conformément à la revendication 18, ce procédé consiste à donner un pH de 1,5 et une température de moins de 60°C au liquide noir dilué.

20. Conformément à la revendication 19, ce procédé consiste à donner une température de 35 à 55°C et une teneur en alcool de 12 à 21% par volume au liquide noir dilué.

21. Conformément à la revendication 14, ce procédé se caractérise par le fait que le liquide noir contient de l'eau avant d'être dilué avec l'acide aqueux retraité.

22. Conformément à la revendication 21, ce procédé consiste à mélanger intimement et rapidement le liquide noir avec l'acide aqueux retraité afin d'obtenir un liquide noir dilué.

23. Conformément à la revendication 15, ce procédé consiste à donner une température de 70 à 95°C au liquide noir dilué et une température de moins de 50°C à l'acide aqueux retraité.

24. Conformément à la revendication 23, ce procédé consiste à donner une température de 80 à 92°C au liquide noir dilué et une température de 25 à 40°C à l'acide aqueux retraité.

25. Un appareil à pulper le bois ou toute autre plante fibreuse avec un solvant organique miscible dans l'eau pour produire un liquide noir comme dérivé et récupérer par la suite le solvant contenu dans le liquide noir, et caractérisé par :

a) une technique de dilution de liquide noir avec un acide aqueux afin d'en précipiter la lignine et obtenir un surnatant dépourvu de lignine ;

b) une technique permettant de séparer par la suite le précipité de lignine du surnatant dépourvu de lignine ;

c) une technique permettant d'extraire par la suite le solvant du surnatant dépourvu de lignine pour obtenir un surnatant dépourvu à la fois de solvant de lignine ; et

d) une technique permettant de retraiter une partie du surnatant dépourvu de solvant et de lignine afin de l'utiliser comme l'acide aqueux employé dans la technique de dilution.

26. Conformément à la revendication 25, cet appareil se caractérise par le fait que la technique de dilution est associée à un système d'extraction d'une partie du solvant du liquide noir avant la dilution de ce dernier.

27. Conformément à la revendication 26, cet appareil se caractérise par l'association de la technique de retraitement avec une technique permettant d'ajouter un acide fort soluble dans l'eau au surnatant dépourvu de solvant et de lignine avant de le retraiter pour l'utiliser dans le système de dilution.

28. Conformément à la revendication 25, cet appareil se caractérise par lu fait que le système de dilution comprend un procédé qui permet de mélanger rapidement et intimement le liquide noir avec l'acide aqueux.

WOOD
CHIPS

FIG.1

ACID
AND
WATER

DILUTED
RESIDUAL
BLACK
LIQUOR TO
SETTLING
TANK 12

RESIDUAL
BLACK LIQUOR
FROM FLASH TANK 11

FIG.2